Europäisches Patentamt

European Patent Office

Office européen des brevets

(11) Publication number: **0 321 604 B1**

(12) **EUROPEAN PATENT SPECIFICATION**

(45) Date of publication of patent specification: **08.07.92**

(21) Application number: **87119130.0**

(22) Date of filing: **23.12.87**

(51) Int. Cl.5: **G01N 33/577**, G01N 33/564, G01N 33/536, G01N 33/541, G01N 33/569, G01N 33/576, G01N 33/80

(54) Antigen-antibody complex and method of using same.

(43) Date of publication of application:
**28.06.89 Bulletin 89/26**

(45) Publication of the grant of the patent:
**08.07.92 Bulletin 92/28**

(84) Designated Contracting States:
**CH DE FR GB IT LI**

(56) References cited:
**EP-A- 0 000 938**     **EP-A- 0 034 050**
**EP-A- 0 074 271**     **EP-A- 0 143 574**
**WO-A-83/01118**     **WO-A-85/04422**
**US-A- 4 529 712**

**CHEMICAL ABSTRACTS, vol. 107, 1987, Columbus,OH (US); J.J.HAAIJMAN et al., p. 528, no. 173765j**

**METHODS IN ENZYMOLOGY, vol. 121, 1986; pp. 548-556**

(73) Proprietor: **SANKO JUNYAKU CO., LTD.**
**TMM Bldg. 10-6 Iwamoto-cho 1-chome**
**Chiyoda-ku Tokyo 101(JP)**

(72) Inventor: **Yamano, Hajime**
**136, Tsutsujigaokakita 6ban-cho**
**Nabari-shi Mie 518-04(JP)**
Inventor: **Nakade, Toru**
**266-6, Haibara-cho sasagaku**
**Uda-gun Nara 633-02(JP)**
Inventor: **Takahashi, Hideo**
**12-41-1204, Nakamoto 5-chome Higashinari-ku**
**Osaka-shi Osaka 537(JP)**
Inventor: **Matsukura, Harumichi**
**3-7-17, Taisho**
**Kashiwara-shi Osaka 582(JP)**
Inventor: **Okubo, Yasuto**
**11-18, Oji-cho taishi 3-chome**
**Kitakatsuragi-gun Nara 636(JP)**

(74) Representative: **TER MEER - MÜLLER - STEINMEISTER & PARTNER**
**Artur-Ladebeck-Strasse 51**
**W-4800 Bielefeld 1(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

The present invention relates to a method of using an antigen-antibody complex.

"Methods in Enzymology", volume 121 (1986) pages 548 to 556 discloses a method of using antiimmunoglobulin coupled to red blood cells in agglutination of monoclonal antibodies, particularly on page 552 and in Table II.

One of the conventional reagents for use in testing the Rh blood type is a monoclonal human anti-D antibody. The production and the use of this monoclonal human anti-D antibody are described in the Proceedings of the 13th Japanese Society for Immunology (1983), pp. 416-417, and Japanese Patent Kokai Nos. 58-500366, 59-138959, and 60-70361. Another reagent is a polyclonal human anti-D antibody obtained from the human serum. This is a commonly used, officially licensed reagent.

For virus detection, a reagent produced by sensitizing or binding sheep red cells with a monoclonal mouse anti-HBs antibody is commonly used in passive hemagglutination.

However, the reactivity of the above monoclonal antibody with the corresponding antigen is so low that the antibody is useful for only a few testing methods. For the Ph blood typing, for example, there are an anti-human globulin method, a bromelin method, an albumin method, and a physiological saline solution method. The monoclonal human anti-D antibody presented in the 13th Japanese Society for Immunology (1983) is able to cause agglutination by the anti-human globulin or bromelin method an determine the blood type of an Rh(D) antigen or saline solution method, thus putting a limit on its applicability.

The polyclonal human anti-D antibody is a human origin reagent using human serum or its dilution with a proper diluent and it is difficult to produce it in large amounts because the human serum is obtained usually by administering Rh negative persons with the Rh positive blood. The monoclonal anti-HBs antibody bound sheep red cell has a low detecting sensitivity or reactivity with the virus antigen.

Accordingly, it is an object of the invention to provide a method of using an antigen-antibody complex useful for various application, especially various blood typing methods.

In attempt to comply with this object, the invention provides a method of using the antigen-antibody complex in which two molecules of a single IgG class monoclonal antibody are bound to one or two molecules of monoclonal or polyclonal anti-IgG antibody specific to the IgG class monoclonal antibody. It reacts with Rh(D) positive blood cells, causing an observable agglutination.

One advantage of the invention is that with the antigen-antibody complex of the invention, an agglutination occurs in a single reaction so that the blood type is able to be determined at once.

Other objects, features, and advantages of the invention will be apparent from the following description when taken in conjunction with the accompanying drawings.

Fig. 1 shows the production of an antigen-antibody complex according to an embodiment of the invention;

FIG. 2 shows agglutination of Rh(D) positive cells by the antigen-antibody complex of FIG. 1;

FIG. 3 shows the production of an antigen-antibody complex according to another embodiment of the invention;

FIG. 4 shows the production of a sheep red cells bound to the antigen-antibody complex of FIG. 3;

FIG. 5 shows passive hemagglutination of HBs antigen by the antigen-antibody complex bound sheep red cells of FIG. 4;

FIGS. 6 and 7 show the use of a conventional monoclonal antibody in an anti-human globulin test;

FIG. 8 illustrates the production of a conventional monoclonal mouse anti-HBs antibody bound sheep red cells; and

FIG. 9 illustrates passive hemagglutination of the HBs antigen with the monoclonal mouse anti-HBs antibody of FIG. 8.

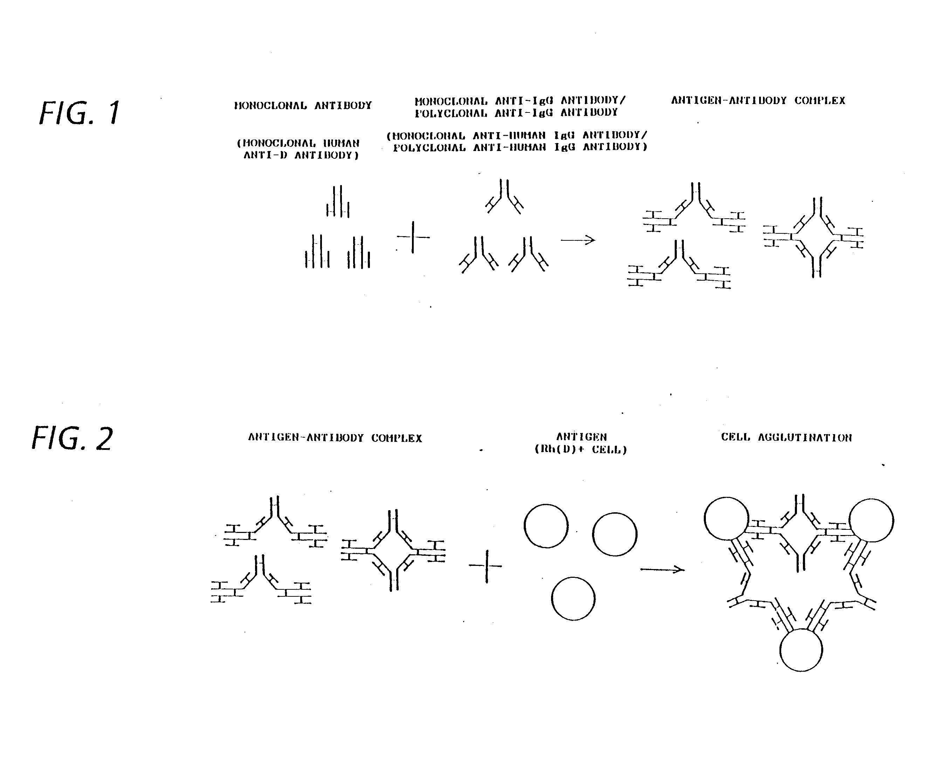

The aforementioned IgG class monoclonal antibody, which has been used for determining the red blood cell surface antigen (blood type substance) or virus antigen, is an antibody against the blood type or virus. According to the invention, the IgG class monoclonal antibody is bound to a monoclonal or polyclonal anti-IgG antibody as shown in FIG. 1. That is to say, an IgG class monoclonal antibody, such as monoclonal human anti-D antibody, and a monoclonal IgG antibody, such as monoclonal anti-human IgG antibody, or a polyclonal anti-IgG antibody, such as polyclonal anti-human IgG antibody, are bound to form an antigenantibody complex in which two molecules of the IgG class monoclonal antibody is bound to one or two molecules of the monoclonal IgG antibody. When this antigen-antibody complex is added to an antigen to be detected, such as an Rh(D) positive blood cell, the binding site of the antigen-antibody complex is bound to the antigen, causing an agglutination as shown in FIG. 2. The agglutination is easy to observe for detection of the antigen.

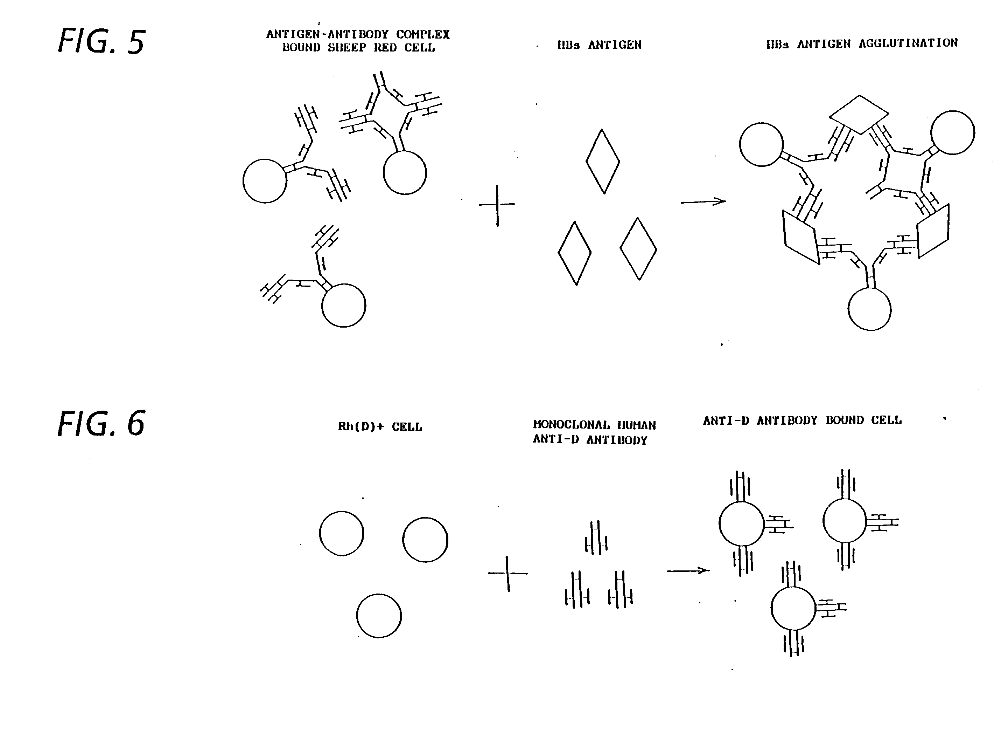

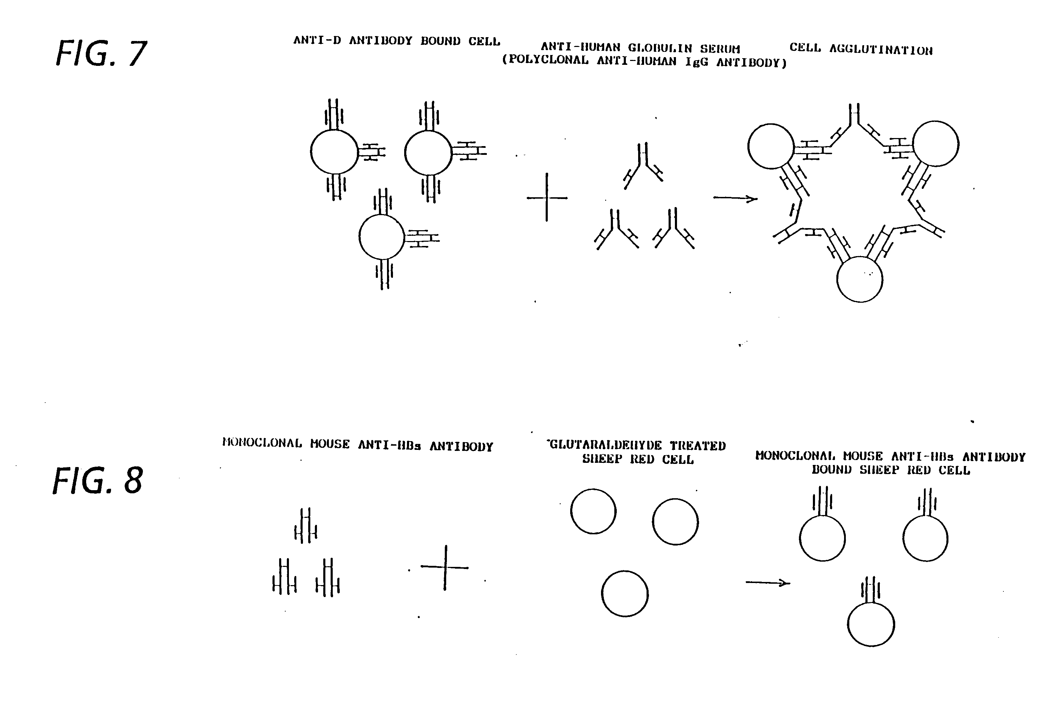

FIG. 6 and 7 show the agglutination of an anti-human globulin test for detecting an RH blood D antigen

2

by using a conventional monoclonal human anti-D antibody. In FIG. 6, a monoclonal human anti-D antibody is added to the Rh(D) positive blood cell and incubated at 37 degrees C for tens of minutes so that the binding site of the monoclonal human anti-D antibody is bound to the Rh(D) antigen or Rh(D) positive blood cell to form an anti-D antibody bound blood cell. Since this is unable to see as an agglutination, an anti-human globulin serum or polyclonal anti-human IgG antibody is added so that the binding site of the polyclonal anti-human IgG antibody is bound to the monoclonal human anti-D antibody, causing an observable agglutination as shown in FIG. 7.

In contrast to the above, when the antigen-antibody complex of the invention is used, an agglutination is formed by only a single reaction so that the blood type is able to be determined at once.

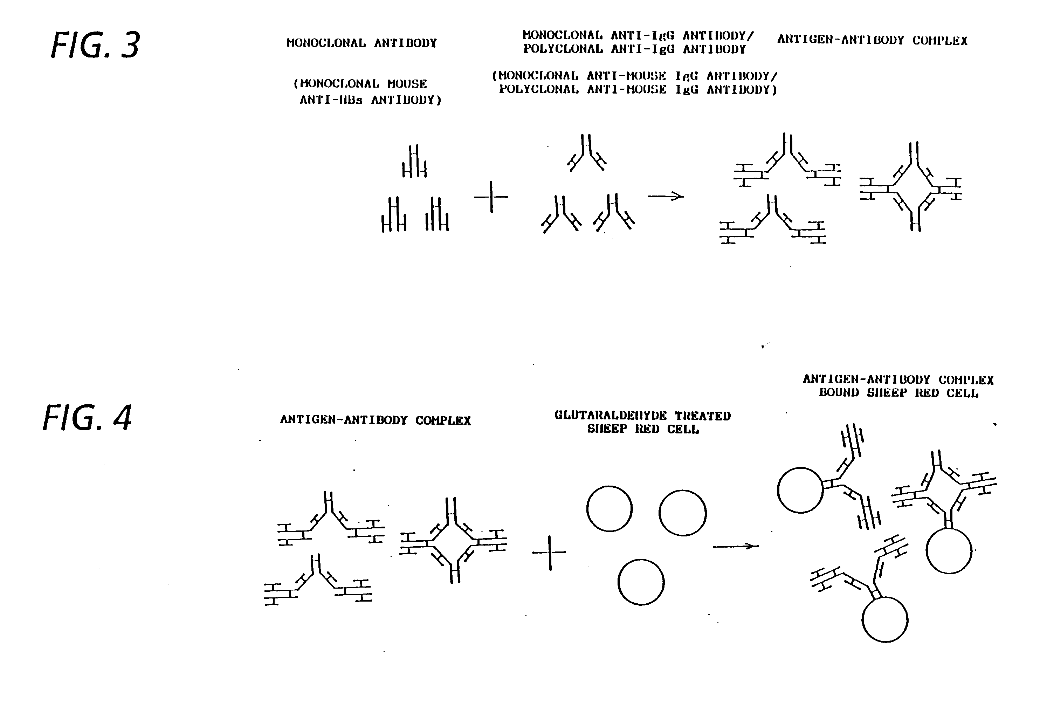

FIGS. 3-5 show the use of the antigen-antibody complex of the invention in passive hemagglutination to detect a virus antigen. In FIG. 3, an IgG class monoclonal antibody, such as monoclonal mouse anti-HBs antibody, reacts with a monoclonal anti-IgG antibody, such as monoclonal anti-mouse IgG antibody, or a polyclonal anti-IgG antibody, such as polyclonal anti-mouse IgG antibody, to form an antigen-antibody complex in which two molecules of the IgG class monoclonal antibody is bound to one or two molecules of the monoclonal or polyclonal anti-IgG antibody.

In FIG. 4, the antigen-antibody complex is bound to sheep red blood cell treated with glutaraldehyde for long shelf life to form an antigen-antibody complex bound sheep red blood cell, which is useful as a virus antigen assay.

In FIG. 5, when this reagent is added to an antigen to be detected, such as an HBs antigen which is a B-type hepatitis virus related antigen, it reacts with the specific antibody or monoclonal mouse anti-HBs antibody, causing an observable agglutination, thus making detection of the antigen possible.

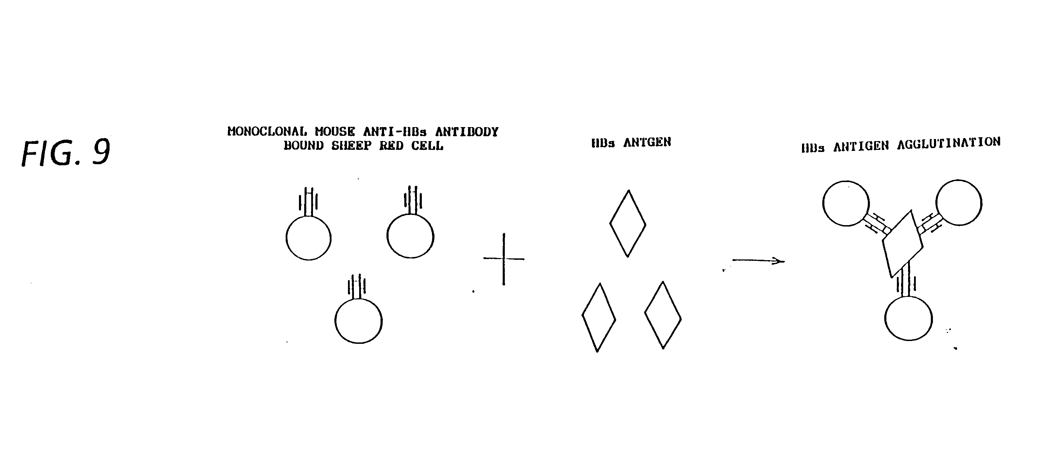

FIGS. 8 and 9 show a conventional passive hemagglutination for detecting an HBs antigen with a monoclonal mouse anti-HBs antibody. In FIG. 8, a monoclonal mouse anti-HBs antibody is bound to the glutaraldehyde treated sheep red cell to form a monoclonal mouse anti-HBs antibody bound sheep red cell, which has been used as an HBs antigen assay. This reagent reacts with the HBs antigen to form an observable agglutination, but its reactivity with the virus antigen is lower than that of the agglutination in FIG. 5.

It is believed that the above agglutination is promoted by the fact that the distance between blood cells is increased by the binding of the antigen, thus decreasing the repulsive force between the blood cells.

EXAMPLE 1

In this example, a monoclonal human anti-D antibody and a monoclonal anti-human IgG antibody are used as an IgG class monoclonal antibody and a monoclonal anti-IgG antibody, respectively.

(1) Production of the Monoclonal Human Anti-D Antibody: Peripheral lymph cells of a Rh(D) negative person with an anti-D antibody were infected with an Epstein-Barr virus (EBV). The transformed cells were cloned in a soft agar culture medium to form a monoclonal human anti-D antibody strain (B2-1 strain). This procedure is described by Masatsune Uno et al. in the Proceedings of the 13th Japanese Society Immunology (1983), pp. 416-417.

The thus cloned B2-1 strain and a JMS-3 strain (human cell) are fused in PEG-1500. The fused cells were cloned by limiting dilution technique to form a monoclonal human anti-D antibody producing strain (B-7-D-10 strain). This anti-D antibody was purified by separating the IgG fraction by a 50% ammonium sulfate method and selecting the IgG class monoclonal human anti-D antibody with 0.0175 Mol phosphate buffered solution (PBS), pH 6.8, using DEAE Sephacell (Pharmacia Co.).

(2) Production of the Monoclonal Anti-Human IgG Antibody: A BALB/C mouse was immunized with the human IgG obtained by purifying human serum with DEAE Sephacell. The harvested spleen cells were fused in PEG-4000 with a P3-X63-Ag8-U1 strain derived from a BALB/C mouse. The fused cells were cloned by limiting dilution technique to form a monoclonal anti-human IgG antibody producing strain according to the method described by Takeshi Watanabe in Immunology Experimental Procedures IX (1978), p 2963. The anti-IgG antibody as purified in the same manner as in (1) above to provide an IgG monoclonal anti-human antibody.

(3) Determination of Titer of the Monoclonal Human Anti-D Antibody: 0.1 ml of the anti-D antibody obtained in (1) above was diluted twice serially with physiological saline solution. A suspension of 2% Rh(D) positive cells in saline was added by dropping to the each dilution for reaction at 37 degrees C for 60 minutes. After the reaction product was washed with saline three times, two drops of anti-human globulin serum was added to it and the mixture was centrifuged at 3400 rpm for 15 seconds. The maximum dilution magnification showing agglutination was taken as the titer.

(4) Determination of Titer of the Monoclonal Anti-human IgG Antibody: 0.1 ml of the anti-IgG antibody obtained in (2) above was diluted twice serially with a diluent containing 3% fetal bovine serum (FBS) and 0.1% $NaN_3$ in saline. A 2% anti-D bound cell saline, which had been prepared by mixing a volume of 2% suspension of $R_1$ and/or $R_2$ cells in saline with a volume of anti-D serum ( albumin antibody) diluted 100 times with saline and incubating the mixture at 37 degrees C for one hour, was added by dropping to the each dilution. The mixture was centrifuged at 3400 rpm for 15 seconds. The maximum dilution magnification showing agglutination was taken as the titer.

(5) Production of the Antigen-Antibody Complex: The monoclonal human anti-D antibody obtained in (1) above was diluted with a 0.013 Mol PBS, pH 7.0, containing 2% bovine albumin and 0.1% $NaN_3$ so that it had a titer of 4096 in the indirect anti-globulin test with Rh(D) positive cells. The monoclonal anti-human IgG antibody obtained in (2) above was adjusted to have titer of 256 as in (4) above. Equal volumes of these dilutions were mixed and incubated at 37 degrees C for one hour to form an antigen-antibody complex in which two molecules of the IgG class monoclonal human anti-D antibody (monoclonal antibody) were bound to one or two molecules of the monoclonal human IgG antibody (monoclonal anti-IgG antibody) specific to the above antibody. See FIG. 1.

(6) Use of the Antigen-Antibody Complex as an Rh Blood Type Assay: The antigen-antibody complex obtained in (5) above reacted with Rh(D) positive cells, showing strong agglutination. See FIG. 2. However, it did not react with Rh(D) negative cells.

Table 1 shows the test results using a conventional monoclonal human anti-D antibody and the antigen-antibody complex of the invention in the saline method. It indicates that the antigen-antibody complex of the invention exhibited a strong agglutination, but the monoclonal human anti-D antibody did not show any agglutination.

Table 2 shows the test results on the same samples as the above using a polyclonal anti-D antibody by the albumin method and the antigen-antibody complex of the invention by the saline method. Both reagents showed strong agglutination with Rh(D) positive cells, indicating the antigen-antibody complex was effective for determining the Rh blood type.

Table 1

| Cells Rh Expression | Cases | Saline Method | |
|---|---|---|---|
| | | MHAD Antibody[1] | A-A Complex[2] |
| Rh(D) positive | 800 | (-) | + + + + |
| Rh(D) negative ccdEE | 7 | (-) | (-) |
| ccdEe | 12 | (-) | (-) |
| Ccdee | 5 | (-) | (-) |
| ccdee | 8 | (-) | (-) |

[1] - Monoclonal human anti-D antibody
[2] - Antigen-antibody complex of the invention

Table 2

| Cells Rh Expression | Cases | Saline Method A-A Complex[1] | Albumin Method MAD Antibody[2] |
|---|---|---|---|
| Rh(D) positive | 800 | + + + + | + + + + |
| Rh(D) negative ccdEE | 7 | (-) | (-) |
| ccdEe | 12 | (-) | (-) |
| Ccdee | 5 | (-) | (-) |
| ccdee | 8 | (-) | (-) |

[1] - Antigen-antibody complex of the invention
[2] - Monoclonal anti-D antibody (Ortho Diagnostic Systems Inc.)

EXAMPLE 2

4

In this example, a monoclonal human anti-D antibody and a polyclonal anti-human IgG antibody were used as an IgG class monoclonal antibody and a polyclonal anti-IgG antibody, respectively.

(1) The production and titer determination of a monoclonal human anti-D antibody were carred out in the same manner as in (1) and (3), respectively, of Example 1.

(2) Production of the Polyclonal Anti-Human IgG Antibody: The IgG franction was separated from normal human serum by a 50% ammonium sulfate method and purified with DEAE Sephacell (Pharmacia Co.). This purified IgG was inoculated into a rabbit. After different agglutinins were absorbed with neuraminidase treated human cells, the IgG fraction was separated from the resulting serum by a 50% ammonium sulfate method and purified with DEAE Sephacell to provide a polyclonal anti-human IgG antibody.

(3) The titer determination and the production of an antigen-antibody complex were carried out in the same manner as in (4) and (5), respectively, of Example 1. In the antigen-antibody complex, two molecules of IgG class monoclonal antibody were bound to one or two molecules of polyclonal anti-IgG antibody. See FIG. 1.

(4) Use of the Antigen-Antibody Complex as Rh Blood Type Assay: The antigen-antibody complex obtained in (3) above reacted with Rh(D) positive cells, showing strong agglutination, while it showed no agglutination with Rh(D) negative cells.

Tables 3 and 4 below show the test results corresponding to Tables 1 and 2, respectively. From these tables, it is apparent that unlike the mere monoclonal human anti-D antibody, the complex of the monoclonal human anti-D antibody and the polyclonal anti-human IgG antibody showed strong agglutinations as in Example 1. See FIG. 1.

Table 3

| Cells Rh Expression | Cases | Saline Method | |
|---|---|---|---|
| | | MHAD Antibody[*1] | A-A Complex[*2] |
| Rh(D) positive | 760 | (-) | + + + + |
| Rh(D) negative ccdEE | 6 | (-) | (-) |
| ccdEe | 12 | (-) | (-) |
| Ccdee | 6 | (-) | (-) |
| ccdee | 8 | (-) | (-) |

*1 - Monoclonal human anti-D antibody
*2 - Antigen-antibody complex

Table 4

| Cells Rh Expression | Cases | Saline Method A-A Complex[*1] | Albumin Method MAD Antibody[*2] |
|---|---|---|---|
| Rh(D) positive | 760 | + + + + | + + + + |
| Rh(D) negative ccdEE | 6 | (-) | (-) |
| ccdEe | 12 | (-) | (-) |
| Ccdee | 6 | (-) | (-) |
| ccdee | 8 | (-) | (-) |

*1 - Antigen-antibody complex of the invention
*2 - Monoclonal anti-D antibody (Ortho Diagnostic Systems Inc.)

This antigen-antibody complex was able to agglutinate in various Rh blood typing methods, such as an anti-human method, bromelin method, and albumin method, as well as a saline method. Also, it was able to use in passive hemagglutination to be described in the following Examples 3 and 4.

EXAMPLE 3

In this example, a monoclonal mouse anti-HBs antibody and a monoclonal anti-mouse IgG antibody were used as an IgG monoclonal antibody and a monoclonal anti-IgG antibody, respectively.

(1) Production of the Monoclonal Mouse Anti-HBs Antibody: A B-type hepatitis virus antigen obtained by purifying an HBs antigen positive serum by a density graduation ultracentrifugal separation method was inoculated into a BALB/C mouse to form a monoclonal mouse anti-HBs antibody producing strain in the same manner as (2) of Example 1. The strain was separated with 4 Mol magnesium chloride by an affinity chromatography in which a purified HBs antigen was bound to Sepharose 4B (Pharmacia Co.) to provide a monoclonal mouse anti-HBs antibody specific to the HBs antigen.

(2) Production of the Monoclonal Anti-Mouse IgG Antibody: A monoclonal anti-mouse IgG rat antibody containing a bovine albumin stabilizer (Immunotech Co.) was used with Bluecelulophine (Biochemical Industry Co.) and a 0.1 Mol trishydrochloric acid buffer solution, pH 8.0, to provide a fraction in which the bovine albumin was bound to the column or an IgG class monoclonal anti-mouse IgG antibody.

(3) Production of the Antigen-Antibody Complex: Equal volumes of the monoclonal mouse anti-HBs antibody of (1) above diluted to a concentration of 10 mg/ml with 0.15 Mol PBS, pH 7.4, by a 280 nm ultraviolet absorption method and the monoclonal anti-mouse IgG antibody of (2) above adjusted to a concentration of 1 mg/ml in the same manner were mixed for reaction at 4 degrees C for 16 hours to produce an antigen-antibody complex. See FIG. 3.

(4) Use of the Antigen-Antibody Complex for Testing HBs Antigen in Passive Hemagglutination:

(a) Production of Bound Cell: Equal volumes of gultaraldehyde treated sheep red cells and the antigen-antibody complex of (3) above or the monoclonal mouse anti-HBs antibody of (1) above were mixed for reaction at 37 degrees C for one hour. The mixture was washed with PBS five times in a centrifuge and suspended in a PBS containing 2% normal horse serum to form a 1% suspension, which was used to produce a bound sheep red cell (bound cell). See FIG. 4.

(b) Use as Assay: It was observed that the reactivity of the antigen-antibody complex bound reagent of (4)(a) was approximately five times higher than that of the conventional monoclonal mouse anti-HBs antibody bound reagent.

Table 5 shows the test results. Human serum containing approximately 5 ng/ml of HBs antigen was diluted 1.5 times on a V-type permanent microplate to make samples. These samples were tested using cells bound to either the conventional monoclonal anti-HBs antibody or the antigenantibody complex of the invention. See FIG. 5.

Table 5

| Dilution of HBs Antigen Positive Serum (HBs antigen content ng/ml) | | | | | | | |
|---|---|---|---|---|---|---|---|
| | x1 (5) | x1.50 (3.33) | x2.25 (2.22) | x3.38 (1.48) | x5.06 (0.99) | x7.59 (0.66) | x11.39 (0.44) |
| *1 | + | ± | - | - | - | - | - |
| *2 | + + | + + | + | + | + | ± | - |
| *1 - Monoclonal Mouse Anti-HBs Antibody Bound Cells | | | | | | | |
| *2 - Antigen-Antibody Complex Bound Cells | | | | | | | |

With the antigen-antibody complex of the invention, agglutination was observed until the HBs antigen was diluted 7.59 times (approximately 0.66 ng/ml) while, with the monoclonal mouse anti-HBs antibody, agglutination was observed until the HBs antigen was diluted 1.5 times (approximately 3.3 ng/ml), indicating that the reactivity of the antigen-antibody complex to the HBs antigen in passive hemagglutination is 5 times higher than that of the monoclonal mouse anti-HBs antobody.

EXAMPLE 4

In this example, a monoclonal mouse anti-HBs antibody and a polyclonal anti-mouse IgG antibody were used as an IgG class monoclonal antibody and a polyclonal anti-IgG antibody, respectively.

(1) Production of the Monoclonal Mouse Anti-HBs Antibody: The monoclonal mouse anti-HBs antibody specific to the HBs antigen was obtained in the same method as in (1) of Example 3.

(2) Production of the Polyclonal Anti-Mouse IgG Antibody: The IgG fraction was separated from BALB/C mouse serm with ammonium sulfate, and the mouse IgG-Fc was purified according to the method described by R. R. Poter in Biochem., 73, p. 119 (1959). A rabbit was immunized with this purified IgG-Fc along with Freund complete adjuvant. The anti-serum obtained from the blood was purified with mouse IgG bound Sepharose 4B to provide a polyclonal anti-mouse IgG antibody.

(3) Production of the Antigen-Antibody Complex: The monoclonal mouse anti-HBs antibody obtained in (1) above was diluted to a concentration of 10 mg/ml with 0.15 mol PBS, pH 7.4, and the polyclonal anti-mouse IgG antibody obtained in (2) above was adjusted to a concentration of 10 mg/ml in the same manner. Equal volumes of these solutions were mixed and reacted at 4 degrees C for 16 hours to produce an antigen-antibody complex. See FIG. 3.

(4) Use of the Antigen-Antibody Complex as HBs Antigen Assay in Passive Hemagglutination:

(a) Production of Bound Cells: Equal volumes of the antigen-antibody complex in (3) above or the monoclonal mouse anti-HBs antibody in (1) above was added to the glutaraldehyde treated sheep red cells and reacted at 37 degrees C for one hour in tannic acid. The mixture was washed with PBS in a centrifuge 5 times and suspended in a PBS containing 2% normal horse serum to form a 1% suspension of bound sheep red cells (bound cells). See FIG. 4

(b) Its Use as Assay: It was observed that the reactivity of the antigen-antibody complex bound reagent obtained in (4) (a) above was approximately 3 times higher than that of the conventional monoclonal mouse anti-HBs antibody bound reagent.

Table 6 shows the testing results. Human serum containing approximately 5 ng/ml HBs antigen was diluted 1.5 times using V-type permanent micro plate to make samples. Tests were conducted by using cells bound to the conventional monoclonal mouse anti-HBs antibody and the antigen-antibody complex of the invention. See FIG. 5.

With the antigen-antibody complex, agglutination was observed until the HBs was diluted 5.06 times (approximately 0.99 ng/ml) while, with the monoclonal mouse anti-HBs antibody, agglutination was observed until the HBs antibody was diluted 1.50 times (approximately 3.3 ng/ml), indicating that the reactivity to the HBs antigen of the antigen-antibody complex of the invention in passive hemagglutination was approximately 3 times higher than that of the monoclonal mouse anti-HBs antibody.

Table 6

| Dilution of HBs Antigen Positive Serum (HBs antigen content ng/ml) | | | | | | | |
|---|---|---|---|---|---|---|---|
| | x1 (5) | x1.50 (3.33) | x2.25 (2.22) | x3.38 (1.48) | x5.06 (0.99) | x7.59 (0.66) | x11.39 (0.44) |
| *1 | + | ± | - | - | - | - | - |
| *2 | + + | + + | + | + | ± | - | - |

*1 - Monoclonal Mouse Anti-HBs Antibody Bound Cells

*2 - Antigen-Antibody Complex Bound Cells

This antigen-antibody complex is useful for latex agglutination as well as the above blood cell agglutination and the passive hemagglutination.

The conventional monoclonal antibody reagent has caused agglutination in only a few blood typing methods and been impractical. By contrast, the antigen-antibody complex of the invention is useful as an Rh blood typing assay for all the blood typing methods by agglutination. In addition, it needs no human serum as polyclonal anti-D antibody so that it is easy to obtain the materials. The antigenantibody complex bound sheep red cells show strong agglutination to a virus, such as an HBs antigen, and have a reactivity approximately 3 to 5 times higher than that of the conventional monoclonal mouse anti-HBs antibody bound sheep red cells.

## Claims

1. Method of using an antigen-antibody complex in which two molecules of a single IgG class monoclonal antibody are bound to one or two molecules of monoclonal or polyclonal anti-IgG antibody specific to said IgG class monoclonal antibody, in agglutination.

2. Method of claim 1, wherein the agglutination is used for blood typing.

3. Method of claim 1, wherein the agglutination is used for virus detection.

## Revendications

7

1.  Procédé d'utilisation d'un complexe antigèneanticorps, dans lequel deux molécules d'un seul anticorps monoclonal de la classe des IgG sont liées à une ou deux molécules d'anticorps monoclonal ou polyclonal anti-IgG, spécifique dudit anticorps monoclonal de classe des IgG, en agglutination.

2.  Procédé selon la revendication 1, dans lequel l'agglutination est utilisée pour la détermination du groupe sanguin.

3.  Procédé selon la revendication 1, dans lequel l'agglutination est utilisée pour la détection virale.

**Patentansprüche**

1.  Verfahren zur Verwendung eines Antigen-Antikörper-Komplexes, in dem zwei Moleküle eines einzelnen Antikörpers der IgG-Klasse In Agglutination verbunden sind mit einem oder zwei Molekülen eines monoclonalen oder polyclonaien Anti-IgG-Antikörpers, der spezifisch ist in bezug auf den monoclonalen Antikörper der IgG-Klasse.

2.  Verfahren nach Anspruch 1, dadurch **gekennzeichnet,** daß die Agglutination verwendet wird für die Blutgruppenbestimmung.

3.  Verfahren nach Anspruch 1, dadurch **gekennzeichnet,** daß die Agglutination verwendet wird für die Entdeckung von Viren.

FIG. 1

MONOCLONAL ANTIBODY

(MONOCLONAL HUMAN
ANTI-D ANTIBODY)

MONOCLONAL ANTI-IgG ANTIBODY/
POLYCLONAL ANTI-IgG ANTIBODY

(MONOCLONAL ANTI-HUMAN IgG ANTIBODY/
POLYCLONAL ANTI-HUMAN IgG ANTIBODY)

ANTIGEN-ANTIBODY COMPLEX

FIG. 2

ANTIGEN-ANTIBODY COMPLEX

ANTIGEN
(Rh(D)+ CELL)

CELL AGGLUTINATION

EP 0 321 604 B1

FIG. 3

MONOCLONAL ANTIBODY

(MONOCLONAL MOUSE
ANTI-HBs ANTIBODY)

MONOCLONAL ANTI-IgG ANTIBODY/
POLYCLONAL ANTI-IgG ANTIBODY

(MONOCLONAL ANTI-MOUSE IgG ANTIBODY/
POLYCLONAL ANTI-MOUSE IgG ANTIBODY)

ANTIGEN-ANTIBODY COMPLEX

FIG. 4

ANTIGEN-ANTIBODY COMPLEX

GLUTARALDEHYDE TREATED
SHEEP RED CELL

ANTIGEN-ANTIBODY COMPLEX
BOUND SHEEP RED CELL

EP 0 321 604 B1

FIG. 5

ANTIGEN-ANTIBODY COMPLEX BOUND SHEEP RED CELL    HBs ANTIGEN    HBs ANTIGEN AGGLUTINATION

FIG. 6

Rh(D)+ CELL    MONOCLONAL HUMAN ANTI-D ANTIBODY    ANTI-D ANTIBODY BOUND CELL

EP 0 321 604 B1

FIG. 7

ANTI-D ANTIBODY BOUND CELL     ANTI-HUMAN GLOBULIN SERUM          CELL AGGLUTINATION
                              (POLYCLONAL ANTI-HUMAN IgG ANTIBODY)

FIG. 8

MONOCLONAL MOUSE ANTI-HBs ANTIBODY      GLUTARALDEHYDE TREATED      MONOCLONAL MOUSE ANTI-HBs ANTIBODY
                                           SHEEP RED CELL              BOUND SHEEP RED CELL

EP 0 321 604 B1

FIG. 9

MONOCLONAL MOUSE ANTI-HBs ANTIBODY
BOUND SHEEP RED CELL

HBs ANTGEN

HBs ANTIGEN AGGLUTINATION

EP 0 321 604 B1